# EUROPEAN PATENT SPECIFICATION

(11) **EP 2 043 612 B1**
(45) Date of publication and mention of the grant of the patent: **28.04.2010**
(21) Application number: 06766006.8
(22) Date of filing: 05.07.2006
(51) Int. Cl.: A61K 9/20, A61K 9/28, A61K 31/4178, A61K 31/5513, C07D 495/04

(54) **A STABLE OLANZAPINE FORMULATION WITH ANTIOXIDANTS**
STABILE OLANZAPIN-FORMULIERUNG MIT ANTIOXIDANTIEN
FORMULATION STABLE D'OLANZAPINE CONTENANT DES ANTIOXIDANTS

(43) Date of publication of application: 08.04.2009
(73) Proprietor: Bilim Ilac Sanayii Ve Ticaret A.S., 34394 Istanbul (TR)
(72) Inventor: FARSHI, Farhad, 34555 Istanbul (TR)
(74) Representative: Berkkam, Ayfer
(86) International application number: PCT/IB2006/052258
(87) International publication number: WO 2008/004033

(56) References cited:
- EP-A- 0 830 858
- WO-A-2004/089313
- US-A- 5 506 248
- US-A- 5 919 485

## Description

This invention is related to stable Olanzapine formulation with antioxidants without subcoating . A stable olanzapine formulation was made by choosing appropriate excipients and production technology.

The compound, 2-methyl-4-4-(4-methyl-1-piperazinyl)-10H-thienol[2,3-b][1,5] benzodiazepine called Olanzapine is an antipsychotic drug substance belongs to thienobenzodiazepine derivatives. Its molecular weight is 312.24. It's a yellow crystalline solid which is practically insoluble in water, sparingly soluble in methanol, soluble in choloroform and easily soluble in dimethylformamid.

Olanzapine is well absorbed and reached peak concentration in approximately 6 hours following an oral dose. It's eliminated extensively by first pass metabolism with approximately 40 % of the dose metabolized before reaching the systemic circulation.

Olanzapine displays lineer kinetic over the clinical dosing range.Its half life ranges from 21 to 54 hours and apparent plasma clearance ranges from 12 to 47 L/hour.

Administration of Olanzapine once daily leads to steady-state concentrations in about one week that are approximately twice the concentrations after single dose.

Olanzapine is extensively distributed throughout the body with a volume of distribution of a approximately 1000 ml. It's 93 % bound to plasma proteins over the concentration range of 7 to 1100 ng/ml

It's known that there are some difficulties of formulating Olanzapine since its sensitivitiy to light and moisture.

For example, in US 2005288276, a stable pharmaceutical formulation is performed by using direct compression. One of the stability problem related with Olanzapine is discoloration of the active substance. They claim that discoloration is caused by Olanzapine hydrates. To avoid from this problem, they prefer making the formulation without using solvents.

In WO 2005009407, a stable pharmaceutical formulation to discoloration is performed by coating Olanzapine with common used excipients called lactose or mannitol.

In US 6190698, Olanzapine Form II is used which is the most stable anhydrous form of Olanzapine. The core formulation is first subcoated with HPMC coating which is free of polyethyleneglycol. The subcoated tablets are coated by mixture of white color (HPMC-PEG-Polysorbate 80-Titaniumdioxide).

In US2001/00232, EP0830858, WO98/13027 HPMC is used for stabilization as a coating agent and granulated with olanzapine . In that patent using of HPMC as coating agent is compulsory to be constituted stable olanzapine tablet. But in our formulation which is subject of this application does not need HPMC. Our invention put forwards using of butylhydroxyanisole without HPMC.

In patent of WO 2004/089313 states obtaining of some stable olanzapine forms and using of butylhydroxyanisole in olanzapine formulation with or without coating. In that patent, butylhydroxyanisole is used in formulation of stable olanzapine forms. For this reason, coating is not sine qua non proviso. However, our invention relates to all of the non-stable olanzapine forms which needs coating.

In order to avoid the stability problems of Olanzapine some various researches were performed which were mentioned above.

Butylhydroxyanisole is used as an antioxidant agent in pharmaceutical formulations. Since it has a function of antioxidant, it can be a solution for active substances which has poor stability. Olanzapine is known as an active substance which has a discoloration problem. This discoloration phenomenia occurs when Olanzapine exposes to air, moisture and light.

It has been found surprisingly that using of antioxidant agent or agents in olanzapine formulation which does not need subcoating and have long term stability . The antioxidant agent is preferably Butylhydroxyanisole. Other suitable antioxidants include but not limited to butylhydroxytoluene, ascorbic acid, propyl gallate etc.

Butyl hydroxyanisole is a widely used antioxidant for long term shelf life of food products, pharmaceuticals and cosmetics by preventing oxidative deterioration of fatty acids (Sang-Hee Jeong, Byung-Yong Kim, Hwan-Goo Kand, Hyun-Ok Ku, Joon-Hyoung Choo, Effects of Butylated Hydroxyanisole On The Development And Functions Of Reproductive System In Rats, Toxicology 208, 2005, page 50, 49-62).

Butyl hydroxyanisole is an antioxidant agent which means to prevent contacting of air to pharmaceutical dosage form. In our invention ,we used Buthylhydroxyanisole in a rate of 0.011 %. Since it has good solubility properties in alcohol.It was dissolved in alcohol (95 % ) and then ,the solution was mixed with distilled water. Finally the granulation solution was completed.

In the known of the art, to reach the stabilization great cellulosic polymers and plasticizers have been used (Farmacia Practica-Remington, 2nd Spanish Edition, Uteha, Mexico 1965, pages 505,506 and 510). Another method is using of subcoating with coating free of plasticizers (EP 0733367). Our invention does not need subcoating for stabilization of olanzapine active ingredient in formulation.

Other advantage of this invention is formulation process takes less time.

The formulation of this invention covers all of forms, polymorphs, salts, solvats etc. of olanzapine.

### Example 1 : Preparation Of Formulation Containing Olanzapine

1. Butylhydroxyanisole is dissolved.
2. Olanzapine and microcrystalline cellulose are granulated with this granulation solution prepared above.
3. The granulated powder mixture is dried
4. Dried granulles are sieved and excipients consisting the exterior phase (which were sieved from 20 mesch) are added.( binder, superdisintegrator, filler, glidant, lubricant )
5. The granulles and powder mixture are mixed .
6. Finally,the lubricant is added to the formulation and mixed.
7. The mixture is compressed with suitable punches in a tablet machine to form core tablets.
8. The core tablets are coated with a polyvinylalcohol based coating material

Coating material is preferably polyvinylalcohol based coating material which is Opadry ® AMB. But other coating materials can be also used.

We use microcrystalline cellulose as a filling material in our formulation. Microcrystalline cellulose derivatives are commonly used in pharmaceutical formulations as filling materials.

To obtain a better dissolution rate ,we need to use a superdisintegrator in our formulation.

In order to improve flow properties of our formulation, in addition to chosing a wet granulation process, we also used a glidant. By using a glidant, we acquire the necessary flowability. As flowability improved better compressibility properties are acquired and we have tablets with no weight variation.

We have to use a lubricant to avoid the adhering of tablets to punches.

In order to acquire the best stability results, in addition to Buthylhydroxyanisole as an antioxidant agent ,we coat our core tablets with a coating material named as Opadry ® AMB white.

There are lots of researches and studies in order to provide the stability of Olanzapine. Some of them mentioned above. These studies were made against the sensitivity of Olanzapine to discoloration, moisture, light.

Differently from other studies, we use Butylhydroxyanisole in our formulation to avoid discoloration of our tablets. Since it is an antioxidant agent and used for preventing air contacting to tablets, discoloration is prevented by means of Butylhidroxyanisole.

Although Olanzapine is known as sensitive to moisture ,we made our formulation by wet granulation and we exposed the active substance to water heat and humidity during drying, we have no stability problems for 40 °C in 6 months, for 30 °C in 12 months, for 25 °C in 24 months. This proves that Butylhydroxyanisole works well in our formulation. Besides Buthylhydroxyanisole ,we also coat our core tablets with Opadry ® AMB white but we need not have subcoated our core tablets as mentioned in EP 0733367 patent. We directly coated tablets ignoring the possibility of any incompatability of coating material with the formulation. Opadry ® AMB white was also protective from moisture.

According to our stability results every parameter investigated in the stability analysises were in limits which were decided according to the ICH (International Community of Harmonization) rules.

## Claims

1. A stable olanzapine formulation with an antioxidant agent and a polyvinylalcohol based coating with the proviso that a subcoating of the formulation is absent.

2. A formulation according to claim 1 wherein the antioxidant agent is butylhydroxyanisole.

3. A formulation according to claim 1 wherein the polyvinyl alcohol based coating is Opadry® AMB.

4. A formulation according to claim 2 wherein the weight of the butylhydroxyanisole is up to 0,01 % of total content.

5. A formulation according to claim 1 wherein the formulation is a solid dosage form.

6. A formulation according to claim 1 wherein the solid dosage form is a tablet.

7. A formulation according to claim 2 wherein the butylhydroxyanisole is treated being dissolved in one or more solvents.

8. A formulation according to claim 7 wherein the solvent or solvents is/are ethyl alcohol and/or water.

9. A formulation according to claim 7 wherein olanzapine and a filing material are granulated with the solvent or solvents.

10. A formulation according to claim 9 wherein to the granulated materials one or more pharmaceutically acceptable excipients selected from the group consisting of a binder, a super disintegrator, a filler, a glidant and a lubricant are added.

11. A formulation according to claim 10 wherein the binder is a cellulose polymer.

12. A formulation according to claim 11 wherein the cellulose polymer is Hydroxypropylcellulose.

13. A formulation according to claim 10 wherein the super disintegrator is selected from the group consisting of Croscarmellose sodium, Sodium Starch Glycolate or Crospovidone.

14. A formulation according to claim 13 wherein the super disintegrator is Sodium Starch Glycolate.

15. A formulation according to claim 10 wherein the filler is made up of cellulose derivatives.

16. A formulation according to claim 15 wherein the cellulose derivatives are Cellactose and/or Microcrystalline cellulose PHIOI.

17. A formulation according to claim 16 wherein the cellulose derivative is Microcrystalline cellulose.

18. A formulation according to claim 10 wherein the glidant is selected from silicium oxide derivatives.

19. A formulation according to claim 18 wherein the glidant is Syloid® 244.

## Patentansprüche

1. Es ist ein stabile Olanzapine Formulierung mit der Bedingung von beinhalten eine Anti- Oxidanten Agent und Verhüllung von eine groben Bedeckung und Polyvinylalkohol basierte material Bedeckung.

2. Es ist eine Formulierung nach Anspruch 1 und die Anti- Oxidanten Agent ist Butyl-Hydroxyianisol.

3. Es ist eine Formulierung nach Anspruch 1 die Polyvinylalkohol basierte Verhüllung ist Opandry ® AMB' dir

4. Es ist eine Formulierung nach Anspruch 2, das beinhaltende Mengengewicht des Butyl-Hydroxyianisol ist mit eine Inhaltsratio vom 0,01 %.

5. Es ist nach Anspruch 1 eine stabile Olanzapine Formulierung mit Anti- Oxidanten und ist in der Form ein festes Pulver.

6. Es ist nach Anspruch 1 eine Formulierung, eine festen Dosis geformte Tablette.

7. Es ist nach Anspruch 2, eine Formulierung in dem der Butyl- Hydroxyianisol in einem oder mehreren Lösemittel gelöst gebraucht wird.

8. Es ist nach Anspruch 7, eine Formulierung das die Lösemittel oder Lösemitteln entweder Äthylalkohol und/ oder Wasser ist.

9. Es ist nach Anspruch 7, eine Formulierung, dass die im Inhalt befindliche Olanzapine und eine Füllungsmaterial mit der Lösematerial oder Materialien gekörnt werden.

10. Es ist im Anspruch 9 beschriebene, den gekrönten Materialien eine bindende, super mahlende, eine Füllungsmaterial, glitschige und ölenden Gruppe ausgewählte ein oder mehreren als Pharmazeutik angenommenen Exipia zugegebene Formulierung

11. Es ist eine Formulierung nach Anspruch 10 und der Bindemittel ist Cellulosen Polymere

12. Es ist eine Formulierung nach Anspruch 11, der Cellulosen Polymer ist Hydroxyd Prophyl Cellulose.

13. Es ist eine Formulierung nach Anspruch 10, der super Mahl ist Crosscamelose Natrium, Natrium Stärke Glykole oder von Crosspovidan gebildeten Gruppe gewählt.

14. Es ist eine Formulierung nach Anspruch 13, der Supermahlmittel ist Natrium Stärke Glykolate.

15. Es ist eine Formulierung nach Anspruch 10, Füllungsmaterial ist aus den Derivaten vom Cellulose gebildet.

16. Es ist eine Formulierung nach Anspruch 15, die Derivaten vom Cellulose sind Cellactose und/ oder Mikrokristall Cellulose PHIOI.

17. Es ist eine Formulierung nach Anspruch 16 die Derivaten von der Cellulose ist Mikrokristalline Cellulose.

18. Es ist eine Formulierung nach Anspruch 10, glitschige Mittel wird aus dem Derivaten vom Zilizium Oxide gewählt.

19. Es ist eine Formulierung nach Anspruch 18, der glitschige Mittel ist Syloid ® 244'tür.

## Revendications

1. Formulation d'olanzapine stable avec agent antioxydant et recouvert d'un matériel à base alcool polyvinylique à condition que la formulation possède un gros revêtement.

2. Formulation selon la revendication 1, **caractérisée en ce que** son agent antioxydant est butylhydroxyanisole.

3. Formulation selon la revendication 1, **caractérisée en ce que** son revêtement à base alcool polyvinylique est Opandry ® AMB.

4. Formulation selon la revendication 2, **caractérisée en ce que** le poids de butylhydroxyanisole dans sa contenu est en ratio de 0, 01 % d'environ du contenu total.

5. Formulation d'olanzapine stable antioxydant selon la revendication 1, **caractérisé en ce qu'**elle est en forme de dose solide.

6. Formulation selon la revendication 1, **caractérisée en ce qu'**elle est un comprimé en forme de dose solide.

7. Formulation selon la revendication 2, dans laquelle butylhydroxyanisole est utilisé en le dissolvant dans un ou plusieurs solvants.

8. Formulation selon la revendication 7, **caractérisée en ce que** ledit solvant ou lesdits solvants sont l'éthanol et/ou l'eau.

9. Formulation selon la revendication 7, **caractérisée en ce qu'**elle est granulée par olanzapine et solvant ou solvants d'une matière emplisseur dans son contenu.

10. Formulation, décrite à la revendication 9, **caractérisée en ce que** un ou plusieurs excipients acceptables pharmaceutiquement sélectionnés du group consistant en un connecteur, super broyeur, une matière emplisseur, surfactant, et lubrifiant.

11. Formulation selon la revendication 10, **caractérisée en ce que** son connecteur est un polymère cellulosique.

12. Formulation selon la revendication 11, **caractérisée en ce que** son polymère cellulosique est l'hydroxypropylcellulose.

13. Formulation selon la revendication 10, **caractérisée en ce que** son super broyeur est choisi du group consistant en croscarmellose sodique, amidon sodique glycolate ou crospovidone.

14. Formulation selon la revendication 13, **caractérisée en ce que** son super broyeur est amidon sodique glycolate.

15. Formulation selon la revendication 10, **caractérisée en ce que** sa matière emplisseur est réalisée en dérivés de cellulose.

16. Formulation selon la revendication 15, **caractérisée en ce que** ses dérivés de cellulose étant sellactose et/ou cellulose microcristalline PHIOI.

17. Formulation selon la revendication 16, **caractérisée en ce que** son dérivé de cellulose est la cellulose microcristalline.

18. Formulation selon la revendication 10, **caractérisée en ce que** son surfactant est choisi parmi les dérivés de l'oxyde de silicium.

19. Formulation selon la revendication 18, **caractérisée en ce que** son surfactant est Syloid ® 224.
